(19) Europäisches Patentamt
European Patent Office
Office européen des brevets

(11) Veröffentlichungsnummer: **0 131 269 B1**

# (12) EUROPÄISCHE PATENTSCHRIFT

(45) Veröffentlichungstag der Patentschrift: **22.03.89**

(51) Int. Cl.⁴: **C 11 D 3/48**, C 07 C 101/26

(21) Anmeldenummer: **84107871.0**

(22) Anmeldetag: **05.07.84**

## (54) Metallchelatkomplexe enthaltende Granulate und Verfahren zu ihrer Herstellung.

(30) Priorität: **12.07.83 DE 3325059**

(43) Veröffentlichungstag der Anmeldung: **16.01.85 Patentblatt 85/3**

(45) Bekanntmachung des Hinweises auf die Patenterteilung: **22.03.89 Patentblatt 89/12**

(84) Benannte Vertragsstaaten: **DE FR GB IT**

(56) Entgegenhaltungen:
**EP-A- 0 012 807**
**EP-A- 0 075 352**
**DE-A- 2 115 081**
**DE-A- 2 141 280**
**DE-A- 2 527 109**
**US-A- 2 936 226**
**US-A- 3 245 776**

(73) Patentinhaber: **BASF Aktiengesellschaft, Carl-Bosch-Strasse 38, D-6700 Ludwigshafen (DE)**

(72) Erfinder: **Jungbauer, Anton, Dr., Schifferstadter Strasse 29, D-6701 Waldsee (DE)**
Erfinder: **Raubenheimer, Hans-Juergen, Benzstrasse 6, D-6834 Ketsch (DE)**
Erfinder: **Leutner, Bernd, Dr., Taunusstrasse 17, D-6710 Frankenthal (DE)**
Erfinder: **Perner, Johannes, Dr., Ginsterweg 4, D-6730 Neustadt (DE)**

EP 0 131 269 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft Granulate, enthaltend einen Metallchelatkomplex der Ethylendiamintetraessigsäure ($H_4EDTA$) in einem Bindemittel und ein Verfahren zur Herstellung dieser Granulate, die beispielsweise aufgrund ihrer bakteriziden und chelatisierenden Wirkung u.a. in Reinigungs- und Desodorierungsmitteln verwendet werden können.

Chelatkomplexe der $H_4EDTA$ mit zweiwertigen und dreiwertigen Metallen, wie Zink, Kupfer, Mangan, Eisen oder Kobalt, in Form ihrer Alkali- oder Ammonium- bzw. Magnesiumsalze sind seit ca. 30 Jahren bekannt. Sie werden in der Regel durch Umsetzung von Ethylendiamintetraessigsäure oder ihren Salzen mit entsprechenden Metallverbindungen hergestellt. In diesem Zusammenhang sei auf die Publikation von St. Chaberek und A. Martell, Organic Sequestering Agents, John Wiley und Sons, New York, 1959, verwiesen, in der auch die vielseitigen Verwendungsmöglichkeiten von Metallchelatkomplexen beschrieben werden.

In den Handel kommen die Komplexverbindungen in reiner fester Form oder als wäßrige Lösungen. Die direkte Verwendung der Stoffe, z.B. als Zwischenprodukte zur Weiterverarbeitung oder als Endprodukte, ist mit einer Reihe von Nachteilen verbunden, weil die reinen Verbindungen stark hygroskopisch sind und deshalb zum Verklumpen und Verkleben neigen. Als Folge davon wird die Transportier- und Dosierbarkeit in ungünstiger Weise herabgesetzt. Die trockenen Pulver, die wegen ihrer hygroskopischen Eigenschaften in der Regel von Luftfeuchtigkeit abgeschlossen gehalten werden, haben den Nachteil, daß bei ihrer Handhabung in trockenem Zustand eine außerordentliche Staubbelästigung auftreten kann. Das Staubproblem wirkt sich dann besonders nachteilig aus, wenn sprühgetrocknete Metallchelatkomplexe verwendet werden, da die durch Sprühtrocknung aus den wäßrigen Lösungen erhaltenen pulverförmigen Chelatverbindungen besonders stark zum Stäuben neigen. Wegen der fertigungstechnischen Vorteile wird jedoch der Großteil der Metall-EDTA-Komplexe über den Weg der Sprühtrocknung hergestellt.

Weiterhin ist aus der DE-A-21 41 280 und DE-A-21 15 081 bekannt, daß Tetranatrium- und Dinatrium-ethylendiamin-tetraacetat zur Verwendung für die Peroxidstabilisierung in Bleich- und Waschflotten mit Fettsäuren oder Paraffin mindestens teilweise eingehüllt werden können.

Die Aufgabe der Erfindung besteht darin, Metallchelatkomplexe der Ethylendiamintetraessigsäure in eine Form zu bringen, die die insbesondere durch Hygroskopizität und Stäuben hervorgerufenen Nachteile nicht mehr aufweisen.

Diese Aufgabe wird gelöst durch Granulate aus einem Metallchelatkomplex der Ethylendiamintetraessigsäure ($H_4EDTA$) und einem Bindemittel, die einen Metallkomplex der Formel

$A_2[M(EDTA)] \cdot x\ H_2O$,
$Mg[M(EDTA)] \cdot x\ H_2O$ oder
$A[M(EDTA)] \cdot x\ H_2O$,

wobei M ein zweiwertiges Metallkation aus der Gruppe Zink, Kupfer und Mangan oder ein dreiwertiges Metallkation aus der Gruppe Eisen und Kobalt, A ein Alkali- oder Ammoniumkation und x eine ganze oder gebrochene Zahl von 0,5 bis 3 bedeuten, in einer Menge von 5 bis 60 Gewichtsprozent in einem bei 25 °C festen Polyethylenoxid, Paraffin, ethoxylierten Fettalkohol oder einer Fettsäure als Bindemittel enthalten.

Die erfindungsgemässen Granulate werden vorteilhafterweise durche eine Schmelzgranulation bei Temperaturen von 60 bis 180 °C hergestellt.

In einer besonders bevorzugten Ausführungsform werden erfindungsgemäße Granulate aus $Na_2[Cu(EDTA)] \cdot xH_2O$ in einer Menge von 5 bis 60, bevorzugt 20 bis 30 Masseprozent in einem ethoxylierten Fettalkohol der Formel

$RO(CH_2CH_2O)_yH$,

in der R einen gesättigten Alkylrest mit 16 bis 18 C-Atomen und y eine ganze Zahl von 11 bis 50 bedeuten, als Bindemittel verwendet.

Die erfindungsgemäßen Granulate, bestehend aus einem der genannten Metallchelatkomplexe und einem Bindemittel, zeigen überraschenderweise auch bei hohen Gehalten an Metallkomplexen eine sehr stark erniedrigte Hygroskopizität und neigen infolge ihrer Teilchengröße mit einem mittleren Durchmesser von 0,5 bis 12 mm, bevorzugt 3 bis 6 mm, nicht mehr zur Staubbildung.

Die starken hygroskopischen Eigenschaften der reinen Metallchelatkomplexe sollen durch einige quantitative Angaben verdeutlicht werden:

Bei einer relativen Luftfeuchte von 65% und 25 °C beträgt die Wasseraufnahme innerhalb von 24 Stunden bei

| | |
|---|---|
| $Na_2[Cu(EDTA)] \cdot H_2O$ | 14,5%, |
| $Na_2[Mn(EDTA)] \cdot H_2O$ | 13,5% und |
| $Na_2[Zn(EDTA)] \cdot H_2O$ | 11,5% |

Als Folge davon zerfließen die festen Chelate an feuchter Luft und werden für bestimmte Verwendungen unbrauchbar.

Die erfindungsgemäß zu verwendenden Bindemittel sind an sich bekannt. Sie werden durch die vorliegende Erfindung erstmals als Bindemittel für Metallchelatkomplexe der Ethylendiamintetraessigsäure verwendet. Sie sind bei Raumtemperatur, d.h. ca. 25 °C fest und schmelzen in der Regel bei Temperaturen unter 180 °C, meist unterhalb von 150 °C. Gegenüber den einzuarbeitenden Metallchelatkomplexen verhalten sie sich inert. Dies sei erwähnt, da eine Reihe von Bindemitteln, die dem Fachmann nahelagen und daher untersucht wurden, diese Voraussetzung nicht erfüllen.

Beispielsweise zersetzt Adipinsäure den Natriumkupferethylendiamintetraessigsäurekomplex unter Gasentwicklung. Andere mögliche Bindemittel, wie Harnstoff, führen nach längerer Lagerzeit zu Farbänderungen und Verbackungen der Granulate.

Die Metallchelatkomplexe der oben angegebenen Formeln sind wasserlöslich. Bei 20 °C können 10 bis 40 masseprozentige wäßrige Lösungen hergestellt werden. Die erfindungsgemäß zu verwendenden Bindemittel sind aufgrund ihrer chemischen Struktur löslich, schwerlöslich oder unlöslich in Wasser. Entsprechend den unterschiedlichen Anwendungszwecken können daher im Hinblick auf die Wasserlöslichkeit der erfindungsgemäßen Granulate die verschiedensten Abstufungen erzielt werden.

Die bevorzugt verwendeten ethoxylierten Fettalkohole, auch Fettalkoholpolyethylenglykolether genannt, sind bekannt und weisen ausgesprochene Tensideigenschaften auf. Sie sind gut wasserlöslich und werden durch Umsetzung von Fettalkoholen mit Ethylenoxid im enstprechenden stöchiometrischen Verhältnis gewonnen. Ihre Zusammensetzung läßt sich durch die allgemeine Strukturformel

$RO(CH_2CH_2O)_yH$,

in der R einen Alkylrest mit 5 bis 30, bevorzugt 16 bis 18 C-Atomen und y eine Zahl von 10 bis 100, bevorzugt 11 bis 50, bedeuten, wiedergeben.

Erfindungsgemäße Granulate mit diesem Bindemitteltyp werden bevorzugt für Wasch- und Reinigungsmittel verwendet.

Beispielhaft sind einige charakteristische Kenndaten von verwendeten ethoxylierten Fettalkoholen in Tabelle 1 zusammengestellt.

Tabelle 1

Kenndaten der ethoxylierten Fettalkohole $RO(CH_2CH_2O)_yH$

| Eigenschaften | Typ 1 | Typ 2 | Typ 3 |
|---|---|---|---|
| Ethoxylierungsgrad y | 11 | 25 | 50 |
| Farbe | gelb | weiß | weiß |
| Schmelzpunkt (°C) | 31 | 40 | 50 |
| pH-Wert (20 °C) (5%ige Lösung in Wasser) | 7,0 | 7,1 | 7,0 |
| Dichte (kg/l; 20 °C) | 0,96 | 1,03 | 1,06 |
| Viskosität (m Pa.s; 60 °C) | ca. 30 | ca. 37 | ca. 39 |

Die erfindungsgemäß zu verwendenden Polyethylenoxide werden durch Umsetzung von Ethylenoxid mit Starterverbindungen wie z. B. Ethylenglykol hergestellt. Als Katalysatoren dienen dabei starke Säuren oder Basen. Die entstehenden Polymerisate zeigen hydrophobe Eigenschaften und lösen sich trotzdem gut in Wasser. Von Säuren und Laugen werden sie kaum angegriffen. Für die Lösung der erfindungsgemäßen Aufgabe eigenen sich die Polyethylenoxide, die bei 25 °C fest sind, d. h. Polyethylenoxid-Typen mit mittleren Molmassen größer ca. 1000, von ca. 1000 bis 500 000.

Im Gegensatz zu den ethoxylierten Fettalkoholen und Polyethylenoxiden sind die als Bindemittel ebenfalls verwendbaren Paraffine und Fettsäuren in Wasser nicht löslich. Bei den Fettsäuren gemäß Anspruch 1 handelt es sich um Verbindungen der allgemeinen Formel $C_nH_{2n+1}COOH$ mit $n \geq 9$. Bevorzugt werden Stearinsäure und Palmitinsäure verwendet; Gemische der Fettsäuren eignen sich ebenfalls.

Die Eigenschaften der als Bindemittel beanspruchten Paraffine können in weiten Grenzen schwanken. In der Regel verwendet man zudem keine reinen Verbindungen, sondern Alkangemische

Die Schmelzpunkte bzw. -bereiche der eingesetzten Paraffine oder Paraffingemische liegen zwischen 45 und 100 °C.

Die Herstellung der erfindungsgemäßen Granulate erfolgt zweckmäßig nach an sich bekannten Methoden durch Schmelzgranulation.

Im einzelnen versteht man unter Schmelzgranulation z. B. Pastillieren, Prillen und Verschuppen, aber auch die Herstellung in Strang- und Lochpressen sowie Extrudieren (Schneckenpressen) kann dazu gerechnet werden, wenn man von schmelzflüssigen Systemen ausgeht.

Nachfolgend soll auf das Pastillieren näher eingegangen werden, weil dieses kontinuierliche Verfahren bevorzugt angewandt wird, um erfindungsgemäße Granulate herzustellen.

Im einzelnen wird das Verfahren folgendermaßen durchgeführt:

Das Bindemittel, z. B. Polyethylenoxid, wird aufgeschmolzen, dann wird der betreffende feste, pulverförmige Metallkomplex zugesetzt und die entstehende Mischung bei der Schmelztemperatur des Bindemittels oder bei höherer Temperatur homogenisiert. Anschließend läßt man die Schmelze, die genau betrachtet ein zweiphasiges Gemisch aus festem Metallkomplex und flüssigem Bindemittel, d. h. eine Dispersion, darstellt, in die Produktverteilerwanne der Pastillieranlage fließen.

Diese besitzt am Boden eine größere Anzahl von Pastillierdüsen, über welchen sich die Pastillierelemente auf- und abwärts bewegen. Als Pastillierelemente werden Nadeln oder Stempel verwendet; die Pastillierdüsen haben gewöhnlich Hohlzylinder- oder Glockenform. Durch die Pastillierelemente wird die Schmelze in Einzelvolumina von etwa Tropfengröße zerteilt und über die Düsen auf ein umlaufendes Band abgestellt.

Das Band kann mit Luft und/oder Wasser gekühlt werden, um das Erstarren der Pastillen zu beschleunigen. Am Ende des Fertigungsprozesses werden die festen, perlen- bis plättchenförmigen Pastillen an der Umlenkstelle des Bandes abgestreift.

Nach einem im Prinzip sehr ähnlichen Verfahren geht man beim Verschuppen vor. Hierbei taucht eine dreh- und kühlbare Walze in die Schmelze des Bindemittels, in dem der Metallchelatkomplex homogen verteilt ist. Nach ca. einer halben bis dreiviertel Umdrehung der Walze streift ein Messer den auf der Walze aufgezogenen und erstarrten Belag in Form von schuppenförmigen Teilchen ab.

Beim Strang- und Lochpressen wird das schmelzflüssige Gemisch aus Bindemittel und

Metallchelat durch eine Düse ausgepreßt und nach Abkühlung mit einer Schneidevorrichtung zerkleinert.

Fertigungstechnisch vorteilhafter gestaltet sich die Verwendung eines Extruders (Schneckenpresse). Dabei werden die beiden Einsatzstoffe (Bindemittel und Metallchelatkomplex) in fester Form der Einzugszone eines Extruders im gewünschten Mengenverhältnis zugeführt. Dort werden die Komponenten miteinander vermischt, in der anschließenden Umwandlungszone aufgeschmolzen, durch die Extruderschnecke verdichtet und durch eine Formdüse ausgepreßt. Die austretenden Stränge werden abgezogen, eventuell zusätzlich gekühlt und von einer Stanzvorrichtung zu zylinderförmigen Granulaten zerteilt.

Man erhält bei Verwendung der erfindungsgemäßen Bindemittel Produkte, deren Metallchelatanteile sich in Wasser langsamer als die reinen Metallchelatkomplexe auflösen. Die Stärke dieses Retardeffekts läßt sich über den Bindemitteltyp und -gehalt in gezielter Weise steuern. Die Stärke dieser Verzögerungswirkung ist bei den wasserunlöslichen Bindemitteln besonders ausgeprägt. Im abgestuften Ausmaß ist sie aber auch bei den wasserlöslichen Bindemitteln vorhanden. Am stärksten tritt dieser Effekt bei Verwendung von Paraffinen auf; die ethoxylierten Fettalkohole zeigen die kleinste Wirkung.

Als Beispiel für die Ausnutzung dieses Retardeffekts sei die Verwendung als Spurenelementdünger aufgeführt. Beim Einsatz der erfindungsgemäßen Granulate für Düngezwecke sind Paraffine und Polyethylenoxide als Bindemittel besonders bevorzugt.

Als anderer Anwendungszweck sei der Einsatz der erfindungsgemäßen Granulate für die Herstellung von Toilettensteinen genannt. Als Bindemittel hierfür eignen sich besonders die ethoxylierten Fettalkohole.

Die Verzögerungswirkung der verschiedenen Bindemittel auf die Auflösung der erfindungsgemäßen Granulate in Wasser wird durch die Versuchsbeispiele in Tabelle 3 veranschaulicht. Zum Vergleich ist auch die Auflösezeit für reines $Na_2[Cu(EDTA)] \cdot H_2O$ und $Na[Fe(EDTA)] \cdot H_2O$ angegeben. Die beiden Verbindungen wurden vor den Versuchen auf einer Zweiwalzenpresse kompaktiert und auf eine mittlere Granulatgröße von ca. 5 mm gebrochen.

Bei der Durchführung der Versuche (vgl. Tabelle 3) wurden jeweils 2 g der entsprechenden Granulate in 100 g Wasser von 25 °C gegeben und die Auflösezeit des Metallchelatanteils bestimmt.

Während des Versuchs wurde mit einem Magnetrührer (1 Umdrehung/s) gerührt. Die Vorteile der erfindungsgemäßen Granulate gegenüber einer Verwendung von reinen Metallchelaten seien nochmals aufgeführt:

– Beseitigung des Staubproblems

– Verringerung der Hygroskopizität der metallkomplexhaltigen Granulate auf 10% der Werte für reine Metallverbindungen

– einstellbare Granulatgröße

– bessere Rieselfähigkeit, Dosierbarkeit und Transportierbarkeit der Granulate verglichen mit pulverförmigen, inbesondere sprühgetrockneten Metallchelatkomplexen

– geringere Anhaftung und Anbackung der Granulate

–steuerbare Retardwirkung über den einstellbaren Bindemittelgehalt.

Hauptanwendungsgebiete der erfindungsgemäßen Granulate sind die Verwendung in Reinigungs- und Desodorierungsmitteln aufgrund der bakteriziden und chelatisierenden Wirkung, daneben werden die Granulate auch als Farbstoffe und als Hilfsmittel in der Galvano- und Färbereitechnik verwendt. Weitere Anwendungsgebiete sind Spurendünger mit verzögerten Nährstoffabgaben, wobei insbesondere wasserunlösliche Bindemittel zweckmäßig sind, und als Katalysatoren bei Polymerisationsreaktionen.

Bei den in den folgenden Beispielen genannten Prozentangaben handelt es sich um Masseprozente.

Beispiel 1

In einem dampfbeheizten 300 l-Rührkessel werden 100 kg ethoxylierter Fettalkohol (Type 3, nach Tabelle 1) aufgeschmolzen und die Temperatur der Schmelze auf 100 °C eingestellt. Danach gibt man 100 kg sprühgetrocknetes $Na_2[Cu(EDTA)] \cdot 2 H_2O$ zu und homogenisiert die Mischung mittels eines Ankerrührers.

Zum Pastillieren läßt man den Ansatz in die beheizte Produktverteilerwanne, einer nach dem Glocken-Stempel-System arbeitenden Pastillieranlage (Hersteller Gebr. Kaiser, Krefeld), fließen. Die Hubzahl, des in der glockenförmigen Düse sich auf und ab bewegenden Stempels, wird dann so eingestellt, daß die abgelegten Tropfen eine möglichst dichte Belegung auf dem mit Wasser gekühlten Band ausbilden. Die optimale Einstellung für die Pastillieranlage ist dann erreicht, wenn die Pastillen an der Abnahmestelle die gewünschte Endtemperatur besitzen und das Band möglichst dicht mit Pastillen der gewünschten Größe belegt ist, ohne daß sie ineinanderfließen. Dies läßt sich außer durch Verstellen der Hubzahl noch durch Verändern von Bandgeschwindigkeit und Bandkühlung erreichen.

Die erhaltenen blaufarbenen Pastillen weisen einen Durchmesser von ca. 6 mm auf. Sie verkleben nicht und zeigen im Unterschied zum reinen sprühgetrockneten $Na_2[Cu(EDTA)] \cdot 2 H_2O$ eine auf 1,2% verminderte Feuchtigkeitsaufnahme (24 Stunden, 65% rel. Luftfeuchte, 25 °C).

Das Produkt ist gut wasserlöslich, läßt sich problemlos lagern sowie dosieren und für Zwecke der Weiterverarbeitung auch wieder zu homogenen Mischungen aufschmelzen.

Beispiele 2 bis 7

Die Beispiele 2 bis 7 werden in Analogie zu Beispiel 1 durchgeführt. Alle wesentlichen Daten, insbesondere Einsatzstoffe und -mengen sowie Pastillierbedingungen und Eigenschaften der erfindungsgemäßen Granulate sind in Tabelle 2 zusammengestellt.

Tabelle 2

Herstellen von Granulaten durch Pastillieren (alle Angaben in Masse-%)

| | Beispiel 1 | Beispiel 2 | Beispiel 3 | Beispiel 4 | Beispiel 5 | Beispiel 6 | Beispiel 7 |
|---|---|---|---|---|---|---|---|
| **Pastillierparameter** | | | | | | | |
| Bindemittel | ethoxylierter Fettalkohol Type 3[1] | ethoxylierter Fettalkohol Type 2[1] | Stearinsäure | Polyethylenoxed mittl. Molmasse ≈6000 | Paraffinge-gemisch (Smp. ≈70 °C) | Paraffin-gemisch (Smp. ≈60 °C | ethoxylierter Fettalkohol Type 3[1] |
| Bindemittelanteil in Pastilliermischung (%) | 50 | 60 | 65 | 55 | 70 | 45 | 75 |
| Metallchelatkomplex | $Na_2[Cu(EDTA)] \cdot H_2O$ | $Na_2[Cu(EDTA)] \cdot H_2O$ | $Na_2[Cu(EDTA)] \cdot H_2O$ | $Na_2[Cu(EDTA)] \cdot H_2O$ | $Na_2[Cu(EDTA)] \cdot H_2O$ | $Na_2[Cu(EDTA)] \cdot H_2O$ | $Na_2[Cu(EDTA)] \cdot H_2O$ |
| Anteil in Pastilliermischung (%) | 50 | 40 | 35 | 45 | 30 | 55 | 25 |
| Temperatur d. schmelzflüssigen Pastilliermischung (°C) | 100 | 75 | 90 | 85 | 85 | 80 | 120 |
| Kühlwassertemperatur (°C) (für Pastillierband) | 8 | 7 | 7 | 7 | 6 | 8 | 9 |
| Bandgeschwindigkeit (m/min) | 4,2 | 6 | 6 | 8 | 5 | 3 | 4,5 |
| Kühlweg (mm) | 5000 | 6000 | 6000 | 5000 | 5000 | 7000 | 5000 |
| innerer Düsendurchmesser ca. (m/m) | 3 | 4 | 5 | 3 | 4 | 4 | 7 |
| Pastillenausstroß (kg/h) | 120 | 100 | 120 | 100 | 130 | 100 | 200 |
| **Pastilleneigenschaften** | | | | | | | |
| Farbe | hellblau | hellblau | hellblau | hellblau | hellblau | beige | farblos |
| Durchmesser, ca. (mm) | 6 | 5 | 6 | 4 | 7 | 7 | 10 |
| Dicke, ca. (mm) | 3 | 2,5 | 3,5 | 2 | 4 | 4 | 5 |
| Schüttdichte (kg/l) | 1,5 | 1,4 | 1,6 | 1,4 | 1,3 | 1,25 | 1,1 |

[1] vergl. Tabelle 1

Tabelle 3

Auflöseverhalten der Metallchelat-haltigen Granulate in Wasser (Retardeffekt)

| Beispiele | Metallchelatanteil | Bindemittelanteil | Granuliermethode | Mittl. Granulat-durchmesser [mm] | Auflösezeit des Metall-chelatanteils |
|---|---|---|---|---|---|
| Vergleichs-beispiel A | 100% $Na[Fe(EDTA)].H_2O$ | – | Kompaktieren | 5 | ≤1 min |
| Vergleichs-beispiel B | 100% $Na_2[Cu(EDTA)].H_2O$ | – | Kompaktieren | 5 | ≤1 min |
| 9 | 50% $Na_2[Cu(EDTA)].H_2O$ | 50% Stearinsäure | Pastillieren | 5 | 24 h |
| 10 | 50% $Na_2[Cu(EDTA)].H_2O$ | 50% ethoxyl. Fett-alkohol (Type 2) | Pastillieren | 5 | 15 min |
| 11 | 50% $Na_2[Cu(EDTA)].H_2O$ | 50% Polyethylenoxid mittl. Molmasse ~6000 | Pastillieren | 5 | 7 min |
| 12 | 50% $Na_2[Cu(EDTA)].H_2O$ | 50% Paraffingemisch (Smp. ~70 °C) | Pastillieren | 5 | 8 h |
| 13 | 30% $Na_2[Cu(EDTA)].H_2O$ | 70% Stearinsäure | Pastillieren | 5 | 48 h |
| 14 | 10% $Na_2[Cu(EDTA)].H_2O$ | 90% Stearinsäure | Pastillieren | 5 | 72 h |
| 15 | 25% $Na_2[Cu(EDTA)].H_2O$ | 75% ethoxyl. Fett-alkohol (Type 2) | Pastillieren | 5 | 30 min |
| 16 | 40% $Na[Fe(EDTA)].H_2O$ | 60% Paraffingemisch (Smp. ~70 °C) | Pastillieren | 5 | 12 h |
| 17 | 40% $Na[Fe(EDTA)].H_2O$ | 60% Polyethylenoxid mittl. Molmasse ~6000 | Pastillieren | 5 | 20 min |
| 18 | 40% $Na[Fe(EDTA)].H_2O$ | 60% ethoxyl. Fett-alkohol (Type 2) | Verschuppen | 5 | 10 min |

Besipiel 8

In einem beheizten 20 l-Kessel werden 5 kg ethoxylierter Fettalkohol (Type 2 nach Tabelle 2) und 5 kg pulverförmiges $Na_2$ [Cu(EDTA)] · $H_2O$ (Wassergehalt 4,5%) vorgelegt und die Mischung unter Rühren auf 120 °C aufgeheizt. Die erhaltene Schmelze leitet man in den beheizten Trog einer Schuppenwalze. Der Durchmesser der verwendeten zylindrischen Walze beträgt ca. 500 mm und die Länge etwa 600 mm.

Die mit Wasser von 8 °C gekühlte Walze rotiert um ihre Längsachse mit einer Umdrehungsgeschwindigkeit von 10 $min^{-1}$. Dabei zieht ein 1 bis 2 mm dicker Film auf, der zusätzlich mit Luft gekühlt wird.

Nach einer Walzendrehung von ca. 240° gemessen von der Oberfläche der Schmelze wird die erstarrte Schicht mit einem Messer abgestreift. Die entstehenden Schuppen von etwa 10 × 10 mm Größe sind gut dosierbar, da sie nicht verkleben oder zu Anhaftungen neigen.

Ihre Feuchtigkeitsaufnahme innerhalb von 24 Stunden bei einer relativen Luftfeuchte von 65% und 25 °C beträgt nur noch 0,9%.

## Patentansprüche

1. Granulate aus einem Metallchelatkomplex der Ethylendiamintetraessigsäure ($H_4$EDTA) und einem Bindemittel, die einen Metallkomplex der Formel

$A_2$[M(EDTA)] · 3 x $H_2O$,
Mg[M(EDTA)] · x $H_2O$ oder
A[M(EDTA)] · x $H_2O$,

wobei A ein Alkali- oder Ammoniumkation, M ein zweiwertiges Metallkation aus der Gruppe Zink, Kupfer und Mangan oder ein dreiwertiges Metallkation aus der Gruppe Eisen und Kobalt und x eine ganze oder gebrochene Zahl von 0,5 bis 3 bedeuten, in einer Menge von 5 bis 60 Masseprozent in einem bei 25 °C festen Polyethylenoxid, Paraffin, ethoxylierten Fettalkohol oder einer Fettsäure als Bindemittel enthalten.

2. Granulate nach Anspruch 1, dadurch gekennzeichnet, daß sie

$Na_2$[Cu(EDTA)] · x $H_2O$

in einem mit 11 bis 50 Mol Ethylenoxid ethoxylierten gesättigten Fettalkohol mit 16 bis 18 C-Atomen als Bindemittel enthalten.

3. Granulate nach Anspruch 1 und/oder 2, dadurch gekennzeichnet, daß sie eine Teilchengröße mit einem mittleren Durchmesser von 0,5 bis 12 mm aufweisen.

4. Verfahren zur Herstellung von Granulaten nach den Ansprüchen 1 bis 2, dadurch gekennzeichnet, daß die Granulate durch Schmelzgranulation bei Temperaturen von 60 bis 180 °C hergestellt werden.

## Claims

1. A granular product composed of a metal chelate complex of ethylenediaminetetraacetic acid ($H_4$EDTA) and a binder, containing a metal complex of the formula

$A_2$[M(EDTA)] · x $H_2O$,
Mg[M(EDTA)] · x $H_2O$ or
A[M[EDTA)] · x $H_2O$,

where A is an alkali metal or ammonium cation, M is a divalent metal cation from the group consisting of zinc, copper and manganese or a trivalent metal cation from the group consisting of iron and cobalt, and x is an integral or fractional number from 0.5 to 3, in an amount of from 5 to 60 weight percent in a polyethylene oxide, paraffin, ethoxylated fatty alcohol or fatty acid binder that is solid at 25 °C.

2. A granular product as claimed in claim 1, which contains

$Na_2$[Cu(EDTA)] · x $H_2O$

in a binder comprising a fatty alcohol of from 16 to 18 carbon atoms ethoxylated with from 11 to 50 moles of ethylene oxide.

3. A granular product as claimed in claim 1 or 2, which has a particle size with an average diameter of from 0.5 to 12 mm.

4. A process for producing a granular product as claimed in any of claims 1 to 3, which comprises producing the granular product by melt granulation at from 60 to 180 °C.

## Revendications

1. Granulés d'un complexe chelaté métallique de l'acide éthylènediamine-tétracétique ($H_4$EDTA) et d'un liant, qui contiennent un complexe métallique de formule

$A_2$[M(EDTA)] · x $H_2O$,
Mg[M(EDTA)] · x $H_2O$ ou
A[M(EDTA)] · x $H_2O$,

A représentant un cation alcalin ou ammonium, M un cation de métal bivalent du groupe zinc, cuivre et manganèse ou cation de métal trivalent du groupe fer et cobalt et x un nombre entier ou fractionnaire de 0,5 à 3, en quantité des 5 à 60% en poids, dans un polyéthylènoxide, paraffine, alcool gras éthoxyle, solide à 25 °C ou un acide gras, comme liant.

2. Granulés selon la revendication 1, caractérisés parle fait qu'ils contiennent

$Na_2$[Cu(EDTA)] · x $H_2O$

dans un alcool gras saturé de 16 à 18 atomes C, éthoxylé avec 11 à 50 moles d'éthylénoxyle, comme liant.

3. Granulés selon la revendication 1 et/ou 2, caractérisés par le fait qu'ils présentent une grosseur de particules d'un diamètre moyen de 0,5 à 12 mm.

4. Procédé de préparation de granulés selon la revendication 1 ou 2, caractérisé par le fait que les granulés sont préparés par granulation par fusion à des températures de 60 à 180 °C.